# EUROPEAN PATENT APPLICATION

(11) **EP 1 645 307 A1**
(43) Date of publication of application: **12.04.2006**
(21) Application number: 05001992.6
(22) Date of filing: 01.02.2005
(51) Int. Cl.: A61N 5/06

(54) **Light source for photodynamic therapy**

(30) Priority: 11.10.2004 IT MI20041923
(71) Applicant: Alpha Strumenti S.r.l., 20133 Milano (IT)
(72) Inventor: Villa, Giuseppe, 20065 Inzago (Milano) (IT)
(74) Representative: Cicogna, Franco

(57) **Abstract**

The present invention relates to a light source for photodynamic therapy with improved characteristics for implementing therapeutic protocols, comprising an emitting unit, which is operatively coupled to a current control device and to a temperature control device.

## Description

### BACKGROUND OF THE INVENTION

The present invention relates to a light source for photodynamic therapy with improved characteristics for implementing therapeutic protocols.

As is known, photodynamic therapy consists of administering a photosensitizing substances and then irradiating by a light source having specifically designed emitting characteristics, depending on the used photosensitizer.

Thus, a localized and selective photochemical reaction causing the death of the tumoural cell is provided.

Also known is the fact that photodynamic therapy (which , will be hereinafter called PDT (PhotoDynamic Therapy) is successfully applied in the therapy of surface tumours and pre-cancerous damages of the skin; however, it has been found as useful for curing tumours of the hollow organs (such as esophagus and bladder).

In recent years, photosensitizers or photosensitizing device having high specificity characteristics for tumoural cells have been increasingly designed.

The above substances (such as ALA - i.e. delta-aminolevunic acid and esterified derivatives thereof) actually consist of ethanol precursors of the photosensitizing substance (IX protoporphyrin - PpIX), which is selectively synthetized in tumoural tissues, owing to the increase of the metabolic rate of tumoural cells with respect to healthy cells.

The administering procedures for administering ALA are mainly of topic nature.

Actually, in particular, ALA is diluted in a water-oil emulsion and then is applied on the tumour involved region.

After a suitable absorption period (2-3 hours), the concentration of the photosensitizing substance (PpIX) in the tumour allow this therapy to be properly performed by irradiating the involved body part with a red light for a period sufficient to achieve the therapeutic light dose.

Tumoular cells containing PpIX in a significative concentration are subjected to a photochemical reaction causing the cells to die because of apoptosis or necrosis.

Healthy cells do not have a significative PpIX concentration, and then they are not damaged.

The above mentioned exposition related to a specific class of photosensitizers, but the disclosed concepts are valid in a general manner.

In fact, each photosensitizer has its characteristic absorption spectrum (which mainly depends on the photosensitizer molecular structure).

The spectrum is indicative of the efficiency with which the light energy or power interact with the molecule (depending on the wavelengths of the impinging light).

Thus, for each photosensitizer, preferential wavelengths allowing a more efficient energy or power transfer to be obtained, are detected.

By way of an example, the absorption spectrum of PpIX (which is synthetized in cells starting from the metabolic precursor ALA) is herein shown.

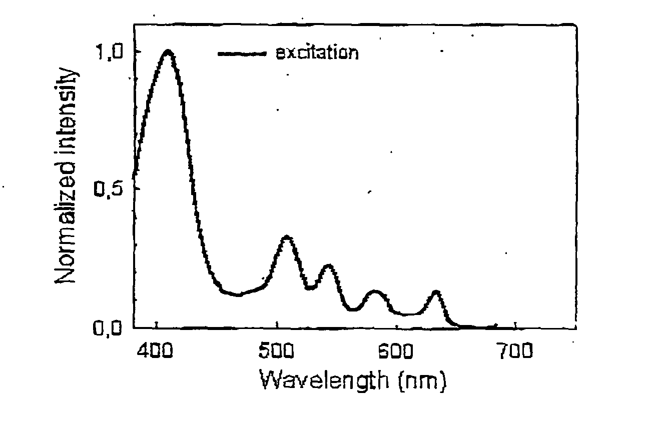

In this diagram, each of the absorption peaks represents an energy transfer efficiency local maximum.

Considering that light radiation is induced to penetrate to a larger depth at greater or longer wavelenghts, the rightmost peak, corresponding to a wavelength of 630 nm is currently used.

At such a wavelength, the penetration into the skin corresponds to about 3 mm (whereas, for example, at a 450 mm peak, which, on the other hand, would provide a greater efficiency), said penetration corresponds to only 0.5 mm.

In the 400-800 mm range, the skin light absorption is mainly determined by hemoglobin and melanin, and this justifies the larger penetration for longer wavelengths (red and infrared).

Upon exposition to light, the photosensitizer is involved into a photochemical reaction generating ROS (reactive Oxygen Species) such as, for example, singlet oxygen.

The above mentioned ROS or reactive oxygen species are susceptible to induce localized cellular damages and, if they are produced in sufficient amounts, they are suitable to induce apoptosis (a programmed cellular death) of tumoural cells.

This mechanism constitutes the basis of a selective therapy against surface tumoural damages or lesions.

Pertinent literature indicates that the efficiency of this mechanism can be overlapped on that of other procedures or methods (such as surgery and chriotherapy methods).

The current practice has found that deltaaminolevulenic acid (ALA) or esters thereof (Methyl-ALA, Benzyl-ALA, Hexyl-ALA) constitute suitable photosensitizers for performing a therapy of cancerous and pre-cancerous lesions of the skin (such as epitheliomas, keratosis, Bowen syndrome).

ALA, as is known, is a homologous component or constituting element of the human body or organism (it pertains to the heme synthesis cycle) and, accordingly, the toxicity thereof is zero.

ALA is topically applied on the skin at the lesion zone, as dissolved in a 5%-20% rate in a water-oil emulsion.

It is not herein necessary to contour or encompass accurately the lesion, since PpIX will be exclusively produced inside the tumoural cells.

After an induction period of 2-3 hours with an occlusive bandaging, the ALA concentration in tissues will arrive at an optimum level.

Under normal conditions, the ALA production rate in the cells is controlled by the action of the enzyme ALA-synthasis.

Upon topically applying ALA, a temporaneous unbalancing of the heme methabolic cycle is caused.

Starting from a larger ALA availability, a greater production and a localized accumulation of protoporphyrin IX, which is a powerful photosensitizer is generated.

The PpIX production rate in tumoural cells is larger than that in healthy cell (depending on the methaboilic rithm alteration in the tumoural cells).

Moreover, tumoural cells provide a greater ALA absorption, depending on the cellular membrane load modifications.

The net or resultant effect is that of concentrating PpIX in tumoural cells, the PpIX rate in healthy cells remaining at a low value.

Then, skin is illuminated with a preset wavelength light (in the case of PpIX by using the 630 nm band or range) for a sufficient period to provide the dose (Joule/cm²) prescribed by the therapeutic protocol.

The clinical practice has been able of experimentally finding the optimum therapeutic doses, depending on the tumour type.

By way of a practical example, are herein shown the "ideal" properties or requirements of a photodynamic therapy light source, as ALA is used (as in the current practice).

Those same properties or requirements would be valid in general for all the photosensitizers, by modifying the parameter values related to each requirement (for example the absorption spectrum):

### Requirement 1:

The source emitting spectrum must be selected to concentrate the source energy or power about the photosensitizer exciting wavelength.

In the case of a photodynamic therapy by ALA, the maximum absorption wavelength compatible with a maximum skin penetration is of about 630+-10 nm.

In the PpIX case, under a threshold of about 50 mW/cm², the production of ROS, as a result of the photosensitizer photochemical reaction, is not sufficient to induce apoptosis in the cell and, accordingly, the tumoural cell, even if it would be damaged, will survive.

Above 200 mW/cm², photo-induced erythemas have been detected (in persons having a very clear skin).

Thus, a lot of authors agree upon an optimum specific power of about 150 mW/cm².

Therapeutic protocols are structured or constructed, as is known, on a series of sessions.

For each session, a light power or energy dose (J/cm²) for irradiating the involved part by the therapeutic light is indicated.

Thus, the observance of the protocol will allow to carry out standardized and reproducible therapy procedures.

In this connection it would be indispensable that the lamp emitting characteristics (the emitting spectrum and specific power) be absolutely reproducible and independent from the environment parameters (for example the room temperature).

### Requirement 2:

The light power must be held as far as possible at a constant value during the emission of the therapeutic dose and the emission must be as constant as possible through the irradiating area.

It is anyhow indispensable that at no place the specific power is caused to be lowered under a minimum level (of about 50 mW/cm²).

By way of example, reference is herein made to the photodynamic therapy which is at present the most diffused one: in other words, that consisting of a topical application of ALA or derivatives thereof and irradiating by a 630 nm source.

It is herein possible to use coherent light sources (the lasers), and in this connection it should be pointed out that the dye lasers can be adjusted to emit in the 630 nm band or range.

On the other hand, a coherent emission has no relevance or significance for the photodynamic therapy purposes.

Considering the comparatively high cost of the above apparatus and the low emission powers (10-20 W) allowing to process a surface of 6-12 cm². such a type of source is not at present used.

In incoherent light sources, on the other hand, the primary source is an incandescent or discharge lamp (for example a Xenon lamp), the emission or emitting spectrum of which is filtered by bandpass optic filters, thereby properly selecting the desired emission or emitting band.

With respect to the power balance, a Xenon lamp source having a power of 750 W, will provide an useful emission of about 100 W.

The main drawback of the above mentioned sources is that are subjected to a degradation of their emitting or emission characteristics, depending on the use time (the so called "lamp ageing").

This will require frequent measurements of the effectively available optical power, in order to be able of holding a constant light energy or power dose.

By way of an example, is hereinbelow shown the emitting or emission spectrum of a halogen lamp for two lamp ages (900 hours and 10,000 hours).

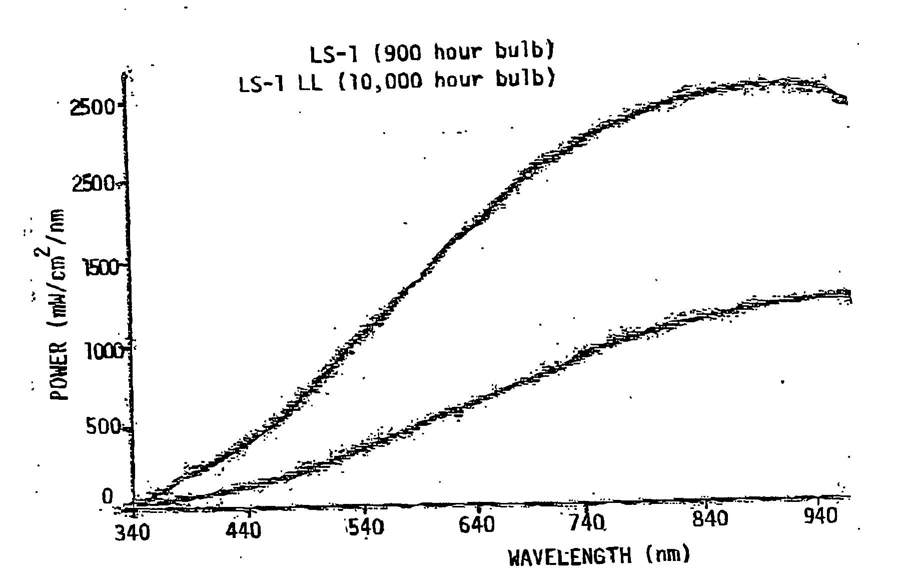

By using solid status sources, such as the light emitting diodes (LED's), emission characteristics near to those of an ideal source will be provided.

By way of an example, is herein shown the emission or emitting spectrum of several LED's.
Relative spektrale Emission^{2) Seite 15}
Relative Spectral Emission^{2) page 15}
V (λ); spektrale Augenempfindlichkeit / Standard eye response curve

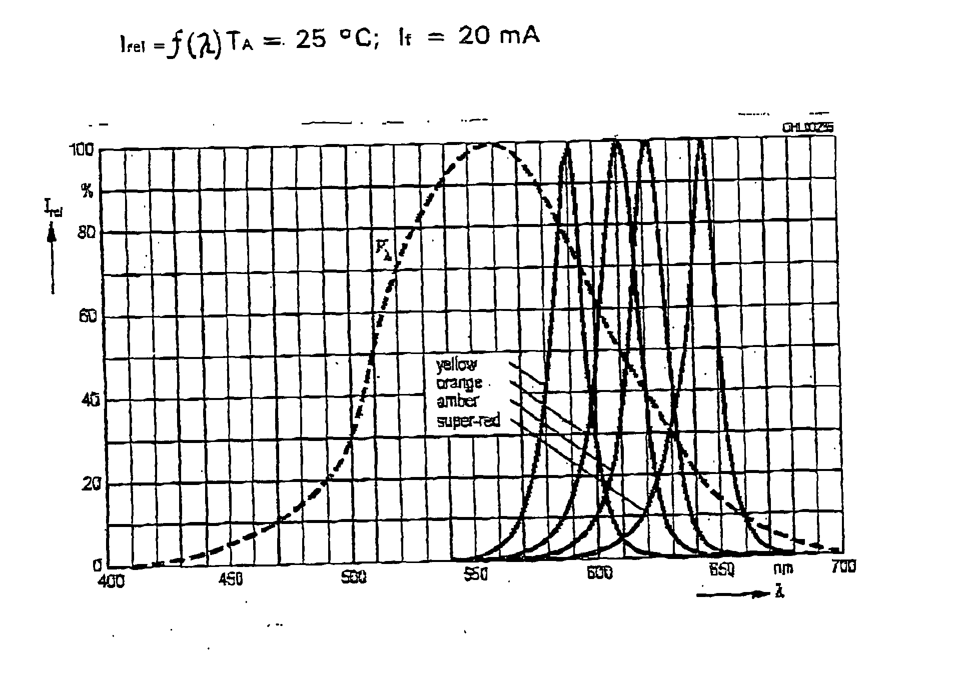

The useful life duration of such a source will be very high [MTBF<100,000] and the deterioration of their characteristics because of ageing is negligible.

The parameters affecting the LED emission (as it will be disclosed in a more detailed manner hereinafter) are:
■ the current passing through the junction
■ the junction temperature

The junction temperature, on the other hand, will depend on the environmental or room characteristics (such as temperature, moisture), in an unpredictable manner).

Several photodynamic therapy light sources based on LED's are commercially available.

However, Applicant submits that none of the above mentioned sources provides control mechanisms on the emitted light doses, depending on the LED junction temperature.

Thus, it would not be possible to provide that constant light emission which would be essential in the herein considered therapeutic protocol.

From a practical standpoint, a source not able of properly controlling the LED junction temperature, will be subjected to the above mentioned negative phenomenon.

Actually, the emitted power will decrease during the emission of the therapeutic doses.

The LED's (embedded in a high thermal resistance plastic holding body) are heated because of the heat generated during their operation.

Thus, a "switching on transitory phenomenon" occurs, causing the optical power to decrease up to 80% during the first operation minutes.

Then the lamp will achieve a thermal balance with a reduced light emission.

However, such a transitory period will depend on the environment conditions (such as the room temperature, lamp temperature and so on) and accordingly, the emitted dose (which is constituted by the time integral of the instantaneous power) could not be reproduced.

### SUMMARY OF THE INVENTION

Accordingly, the aim of the present invention is to overcome the above mentioned drawbacks, by providing a light source which is not subjected to a degradation of its emission or emitting characteristics, depending on its use time, while always providing a substantially constant emission.

Within the scope of the above mentioned aim, a main object of the present invention is to provide such a light source in which it is possible to perform an efficient control of the emitted light dose, depending on the LED junction temperature, thereby preventing the emitted power from decreasing during the emission of the therapeutic dose.

Another object of the present invention is to provide such a photodynamic therapy light source, having improved characteristics for implementing therapeutic protocols which, owing to its specifically designed constructional feature, is adapted to provide a very reliable and safe operation.

Yet another object of the present invention is to provide such a light source which can be easily made and which, moreover, is very competitive from a mere economic standpoint.

According to one aspect of the present invention, the above mentioned aim and objects, as well as yet other objects, which will become more apparent hereinafter, are achieved by a light source for photodynamic therapy with improved characteristics for implementing therapeutic protocols, characterized in that said light source comprises an emitting unit, operatively coupled to a current control device and a temperature control device.

### BRIEF DESCRIPTION OF THE DRAWINGS

Further characteristics and advantages of the present invention will become more apparent hereinafter from the following disclosure of a preferred, though not exclusive, embodiment of a light source for photodynamic therapy with improved characteristics for implementing therapeutic protocols, which is illustrated, by way of an indicative but not limitative example, in the accompanying drawings, where:

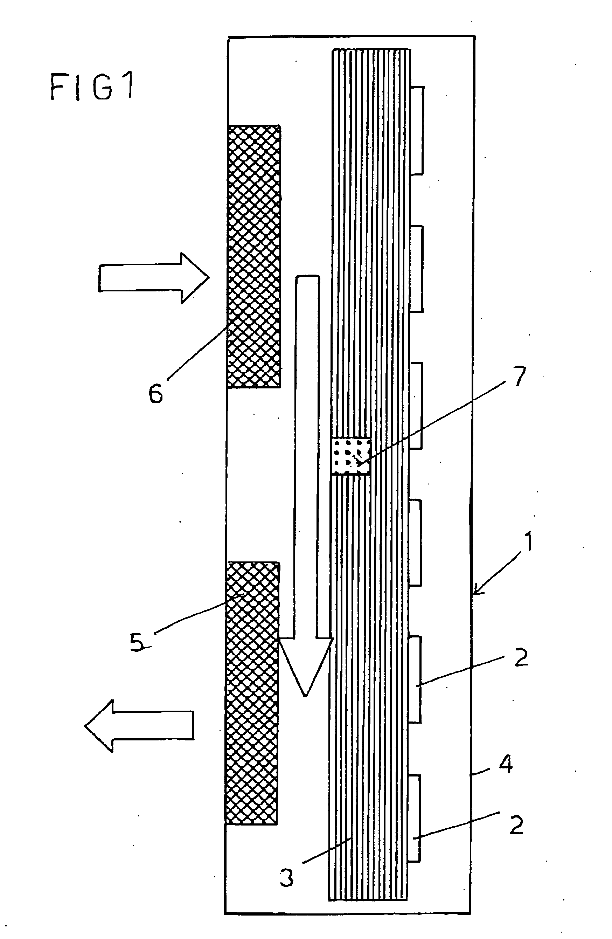

Figure 1 is a schematic view illustrating the emission or emitting unit;
Figure 2 shows the LED supporting heatsink;
Figure 3 shows a diffuser element arranged in front of the LED,

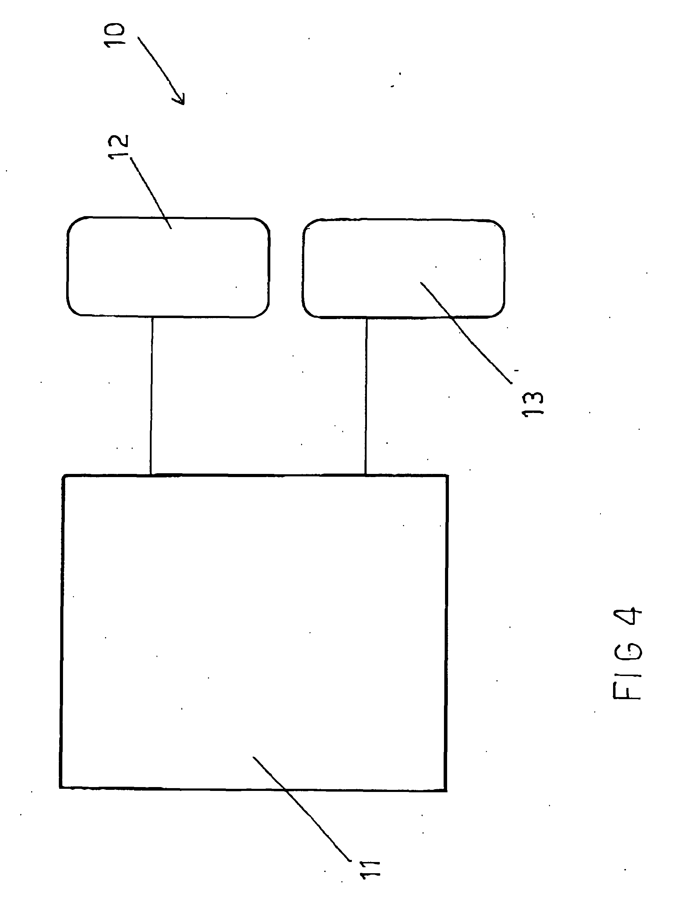

Figure 4 shows a schematic diagram of the current control device; and

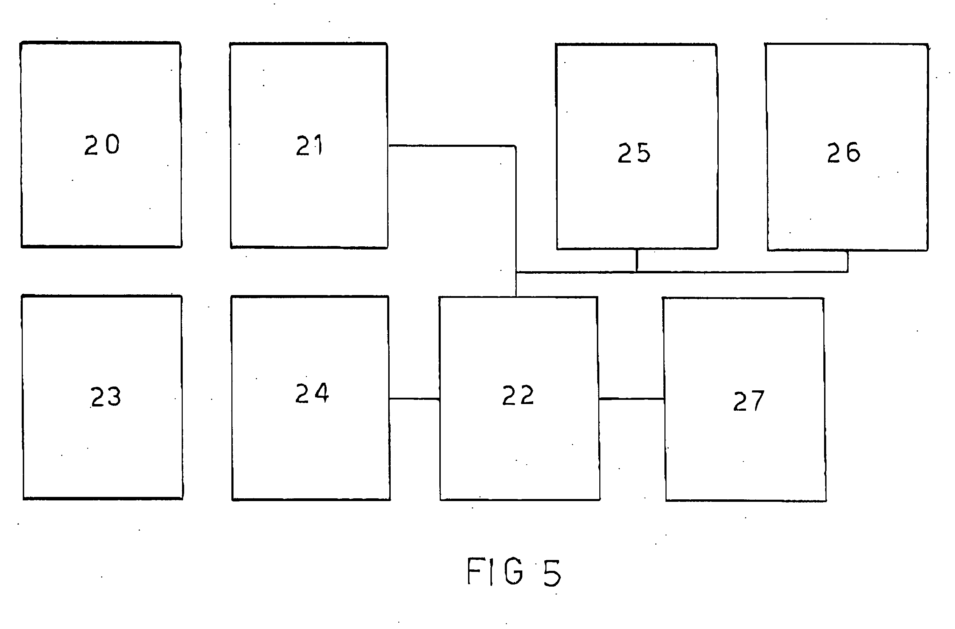

Figures 5 shows the construction of the power supply and control unit.

### DESCRIPTION OF THE PREFERRED EMBODIMENT

With reference to the above mentioned figures, the light source for photodynamic therapy according to the present invention is based on the use of high power light emitting diodes.

The embodiment herein disclosed provides a light emission in the 630 nm range or band, since the photodynamic therapy in the status of the art requires that this wavelength (for using ALA or derivatives thereof) be used.

The novel features of the invention, however, go away from this detail, and they can also be applied for emissions in other bands or ranges (if in a near future novel or new photosensitizer would be available having different exciting or excitation bands).

Differently from the available prior models, both the operating parameters controlling the light emission of the LED are herein controlled and, more specifically:
■ the current
■ the junction temperature

More specifically, the current control is performed by a constant DC power supply; a feedback circuit will hold the junction current at a constant value (i.e. at a preset value).

The junction temperature control, on the other hand, is carried out by a specifically designed LED assembling mechanical arrangement, and by using a forced ventilation.

A LED temperature detecting circuit will allow to compensate for the light energy or power emission, depending on the junction thermal curve.

As it will be disclosed in a more detailed manner hereinafter, the LED diodes have their emitted optical power which is related with a substantially linear relationship with the junction temperature.

Actually, each LED makers will provide the mentioned power/temperature curve which, in each case, can also be experimentally found.

In particular, the LED's are mounted on a metal heatsink, in which a digital temperature sensor is also arranged.

The heatsink temperature will be related to the LED junction temperature, through a characteristic curve, which depends on the mechanical arrangement or construction of the device and which is experimentally measured.

The compensation of the emitted power is made as follows:
- Step 1: the metal heatsink temperature is detected by a temperature digital sensor (accuracy ± 0.5°C)
- Step 2: the LED junction temperature is calculated based on the device characteristic curve
- Step 3: the emitted optical power is then calculated
- Step 4: the contribution of the instantaneous optical power to the total dose being finally calculated.

The above mentioned process is repeated with a constant rate (about each 10 s).

As the total dose achieves the dose value set by the user, the LED's are switched off.

This control is performed by a programmable microswitch, designed for performing the calculations of the operating steps 2, 3 and 4.

In such a procedure, the user will set, through a control panel, the required or target light dose.

This dose will be referred to a specific distance of the lamp from the body part to be irradiated.

With respect to the environmental conditions, the dose is considered as referred to a "cold" lamp, i.e. with a led junction temperature of 25°C, in a thermal equilibrium condition with the encompassing environment.

Under the control of the user, the lamp will be switched on, and the control circuit will detect during the operation the instantaneous emitted power.

Upon achieved the target or set light dose, the lamp will be switched off.

The emission duration or period will depend on the environmental conditions (room temperature and moisture) time period from the last switching on and so on).

The emission or emitting unit 1 comprises:

LED's 2 embedded in a metal-ceramic envelope, providing a low thermal resistance between the diode junction and its envelope or container.

The LED's are assembled on a metal heatsink 3, through the interposition of a heat conductive compound.

The rear part of the above mentioned heatsink is provided with a plurality of longitudinally extending fins.

More specifically, the heatsink is enclosed in a metal enclosure 4, also providing mechanical support and cooling air flow guiding functions.

Inside said envelope 4, fans 5 and 6 will provide a set longitudinal air flow, parallel to cooling fins.

The fan 5 will blow air into the inside of the envelope or vessel, whereas the fan 6 will suck air from said envelope.

The temperature sensor 7 is assembled in a blind hole formed in the heatsink 3.

The thermal contact is assured by a heat conductive compound or material.

Thus, the temperature sensor will detect the temperature at the centre of the heatsink, which temperature will be related to the LED junction temperature through a characteristic curve, which is experimentally constructed, thereby providing the temperature control device.

The light strength or intensity emitted by a LED will substantially depend on the following parameters:

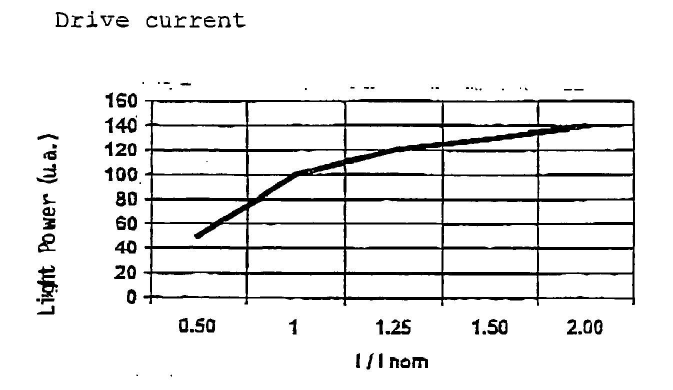

The above graph shows the light emission, depending on the drive current (I) and nominal or reference drive current I nom ratio.

The light emission is represented by an arbitrary optical power unit.

The nominal or reference drive current corresponds to that related to an output optic power of 100 at 25°C.

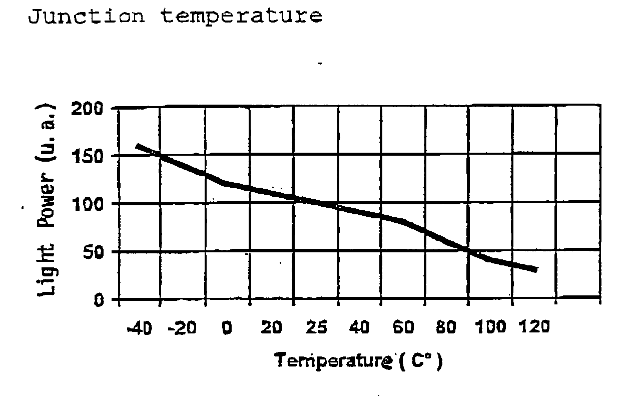

The above curve is supplied by the LED maker, and can be experimentally constructed by using a thermometer and an optical power measuring device.

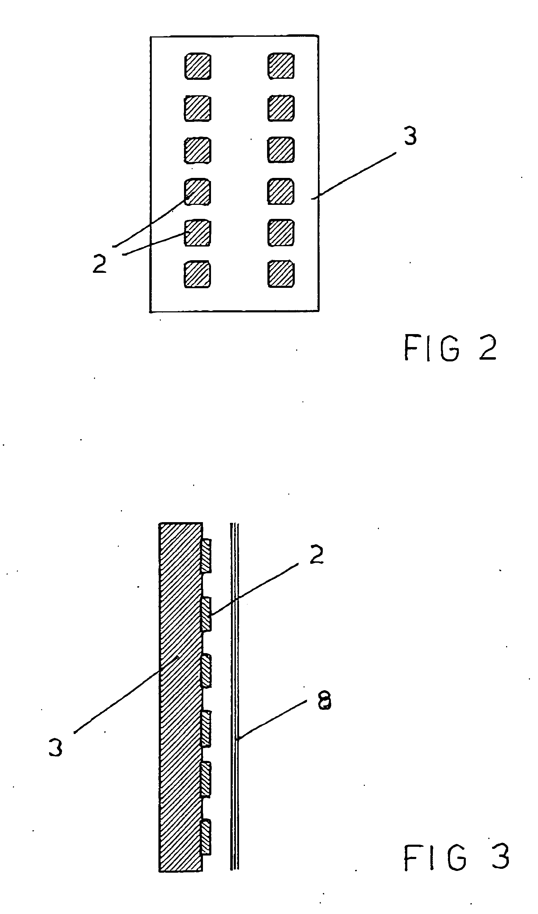

As is shown in figure 2, the LED's 2 are mounted on the heatsink according to an array arrangement (in the shown example 2x3 array arrangement).

The number of the used LED's will depend on the specific power of each emitter.

To provide a homogeneous light power, a microprismatic diffusing element or diffuser 8 is arranged in front of the LED diodes.

The above microprismatic diffuser elements will concentrate the light energy into a cone of about 20°, according to the axis normal to the surface.

The current control device 10 comprises a power supply and control unit 11, including an user interface constituted by an alphanumeric keyboard having 16 keys and an alphanumeric LCD display, as well as one or two irradiating units providing the desired light emission.

The above mentioned power supply unit is coupled to the 220 AC mains or network.

The irradiating units, in turn, are coupled to the power supply unit through a coupling cable (of about 2 m of length) including connectors allowing it to be easily and quickly coupled.

The power supply unit is designed for supplying up two irradiating units 12 and 13, and the use can set a desired unit or both units to be active.

The operating parameters of the apparatus which can be set by the user are the following:
- he irradiating or irradiation dose
- the number of the irradiating units to be simultaneously energized.

The power supply and control unit comprises the following functional blocks:

An unregulated power supply 20 (a rectified bridge + a filtering capacity), designed for supplying 15A at a voltage of 44V.

A constant current power supply 21, designed for supplying the LED assemblies with a constant current.

It is divided into four like or identical benchs provided for supplying each an assembly of 6 LED's arranged in a series relationship.

The maximum absorption or drain of each bench or group is of about 2800 mA.

Each bench comprises trimmer adjuster or adjusting elements for setting the supplied current value: two current supplying levels are herein provided:
- I_nom which corresponds to the nominal or rated emission power of the irradiating elements.
- I_reduced which corresponds to the light guide emission of the irradiating elements.

The control unit 22, by using digital signals, will control the current supply to one or the other irradiating elements, by driving specifically provided relays.

The power supply unit 23, controlled or set at 5V and 12V will supply the voltages of 5V and 12V, respectively, as required for operating the control units.

The adjustment is performed by built-in adjusting elements, of a not controllable or adjustable type which are protected against over voltages and shorts.

The estimated drain for the 5V line is 500 mA.

The estimated drain for the 12V line is 200 mA.

The unstabilized power supply unit 24 (rectified bridge + filtering capacity) is designed for providing 2° at a voltage of 24V.

It is designed for power supplying the cooling fans (both those arranged inside the power supplying and control unit and those arranged on the irradiating elements).

The inner fans are continuously power supplied.

The fans arranged on the irradiating element, on the contrary, will be actuated only as the LED's are switched on and will be switched off after a period sufficient for cooling the irradiating elements.

A digital signal from the control unit will independently switch on the fans of the irradiating elements, by controlling dedicated relays.

The 16 key array keyboard is interconnected to the output of the control unit 22, and cooperates with a liquid crystal display 24 back illuminated by back-illuminating LED's.

It is provided for displaying two array rows having 20 characters each.

By suitable trimmers, it is possible to adjust the back illuminating intensity and the display contrast.

The control unit 22 comprises a programmable microcontroller of the Microchip-PIC® family, and controlled peripheral unit interfacing circuitry.

The designed firmware will implement the operating functions of the device and will manage the user interface (keyboard + LCD).

To the control unit 22, a digital thermometer 27, arranged inside each irradiating element, is associated; if said digital thermometer is interrogated by the control unit, it will transmit a temperature in the °C - 125°C range with an accuracy of 0.5°C.

The control unit 22 is based on a Microchip-PIC® 16F877 microcontroller.

This device comprises, in total, 28 I/O lines which can be managed by a dedicated program, directly interfacing with a lot of interconnected peripheral units.

The program store or memory capability is of 8K bytes; 256 bytes of RAM and 256 bytes of EEPROM being available.

In particular, the microcontroller is programmed by the MPLAP© IDE development system, provided by the MicroChip.

From the above disclosure it should be apparent that the invention fully achieves the intended aim and objects.

In fact, the invention provides a light source for photodynamic therapy having improved characteristics for implementing therapeutic control or protocols, allowing to optimize all the therapeutic parameters, since its current and temperature are simultaneously controlled.

Thus, the inventive source will provide a constant like doses during the therapy procedure.

The invention, as disclosed, is susceptible to several modifications and variations, all of which will come within the scope of the invention.

Moreover, all the constructional details can be replaced by other technically equivalent elements.

In practicing the invention, the used materials, as well as the contingent size and shapes, can be any, depending on requirements.

## Claims

1. A light source for photodynamic therapy with improved characteristics for implementing therapeutic protocols, **characterized in that** said light source comprises an emitting unit, operatively coupled to a current control device and a temperature control device.

2. A light source, according to the preceding claim, **characterized in that** said emitting or emission unit comprises LED's embedded in a metal-ceramic envelope with a low thermal resistance between the diode junction and said envelope.

3. A light source, according to the preceding claims, **characterized in that** said LED's are arranged on a metal heatsink, with the interposition of a heat conductive material.

4. A light source, according to one or more of the preceding claims, **characterized in that** said heatsink comprises longitudinal extending fins arranged at the rear thereof.

5. A light source, according to one or more of the preceding claims, **characterized in that** said light source comprises moreover, inside said metal envelope, an inlet air fan for supplying air to the inside of said envelope and a further fan for sucking air from said envelope.

6. A light source, according to one or more of the preceding claims, **characterized in that** said light source comprises moreover a temperature sensor, arranged in a blind hole formed in said heatsink.

7. A light source, according to one or more of the preceding claims, **characterized in that** said light source comprises moreover, in front of said LED's, a microprismatic diffusing element.

8. A light source, according to one or more of the preceding claims, **characterized in that** said current control device comprises a power supply and control unit including an user interface and coupled to at least a light emitting irradiating source.

9. A light source, according to one or more of the preceding claims, **characterized in that** said power supply and control unit is coupled to two irradiating units.

10. A light source, according to one or more of the preceding claims, **characterized in that** said light source comprises moreover a constant current power supply unit for power supply groups of said LED's .

11. A light source, according to one or more of the preceding claims, **characterized in that** said light source comprises an unregulated power supply unit which is operatively coupled to a control unit.

12. A light source, according to one or more of the preceding claims, **characterized in that** said control unit comprises a programmable microcontroller, operatively associated with a keyboard and a LCD display and connected to a digital thermometer.

13. A light source for photodynamic therapy with improved characteristics for implementing therapeutic protocols, according to one or more of the preceding claims, and substantially as disclosed and illustrated and for the intended aim and objects.
